(19) **Europäisches Patentamt / European Patent Office / Office européen des brevets**

(11) **EP 4 434 621 B1**

(12) ## EUROPEAN PATENT SPECIFICATION

(45) Date of publication and mention of the grant of the patent:
**29.04.2026 Bulletin 2026/18**

(21) Application number: **23163031.0**

(22) Date of filing: **21.03.2023**

(51) International Patent Classification (IPC):
*B01J 23/755* (2006.01)    *B01J 21/16* (2006.01)
*B01J 19/08* (2006.01)    *C07C 1/12* (2006.01)
*C10L 3/08* (2006.01)    *C01B 3/08* (2026.01)
*B01J 8/02* (2006.01)    *B01J 35/45* (2024.01)
*B01J 35/61* (2024.01)    *B01J 35/63* (2024.01)
*B01J 35/30* (2024.01)

(52) Cooperative Patent Classification (CPC):
(C-Sets available)
**B01J 23/755; B01J 8/02; B01J 19/088; B01J 21/16; B01J 35/393; B01J 35/45; B01J 35/618; B01J 35/638; C01B 3/08; C07C 1/12; C10L 3/08;** B01J 2219/0847; B01J 2219/0896; B01J 2235/15; C07C 2523/755;    (Cont.)

(54) **METHOD AND SYSTEM FOR PLASMA-CATALYTIC CARBON DIOXIDE HYDROGENATION WITH IN-SITU WATER REMOVAL AND ADDITIONAL HYDROGEN PRODUCTION**

VERFAHREN UND ANLAGE ZUR PLASMAKATALYTISCHEN KOHLENDIOXIDHYDRIERUNG MIT IN-SITU-WASSERENTFERNUNG UND ZUSÄTZLICHER WASSERSTOFFERZEUGUNG

PROCÉDÉ ET SYSTÈME D'HYDROGÉNATION CATALYTIQUE AU PLASMA DE DIOXYDE DE CARBONE AVEC ÉLIMINATION IN SITU DE L'EAU ET PRODUCTION SUPPLÉMENTAIRE D'HYDROGÈNE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**25.09.2024 Bulletin 2024/39**

(73) Proprietor: **Lietuvos Energetikos Institutas**
**44403 Kaunas (LT)**

(72) Inventors:
• **TAMOSIUNAS, Andrius**
**53458 Virbaliskiai, Kauno r. sav. (LT)**
• **GIMZAUSKAITE, Dovile**
**09206 Vilnius (LT)**
• **AIKAS, Mindaugas**
**45311 Kaunas (LT)**
• **USCILA, Rolandas**
**44461 Kaunas (LT)**
• **LUKOSIUTE, Stase Irena**
**46299 Kaunas (LT)**
• **MILCIUS, Darius**
**51310 Kaunas (LT)**

• **VARNAGIRIS, Sarunas**
**49286 Kaunas (LT)**
• **URBONAVICIUS, Marius**
**54306 Vijukai, Kauno r. sav. (LT)**
• **STRIUGAS, Nerijus**
**54354 Domeikava, Kauno r. sav. (LT)**

(74) Representative: **Pranevicius, Gediminas**
**Law firm IP Forma**
**Uzupio g. 30**
**01203 Vilnius (LT)**

(56) References cited:
**EP-A1- 2 650 401    CN-A- 107 413 346**

• **ASHOK JANGAM ET AL: "A review of recent catalyst advances in CO2 methanation processes", CATALYSIS TODAY, ELSEVIER, AMSTERDAM, NL, vol. 356, 29 July 2020 (2020-07-29), pages 471 - 489, XP086304345, ISSN: 0920-5861, [retrieved on 20200729], DOI: 10.1016/J.CATTOD.2020.07.023**

EP 4 434 621 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
C10L 2290/38

C-Sets
**C07C 1/12, C07C 9/04**

**Description**

FIELD OF THE INVENTION

The present invention relates to the field of synthetic methane production with *in-situ* produced water/water vapour removal and additional hydrogen generation from it. In particular, the present invention relates to synergetic effect of a gliding arc discharge plasma (GADP) and catalytic carbon dioxide hydrogenation with subsequent produced water removal and additional hydrogen production at low temperatures and atmospheric to moderate-level pressures.

BACKGROUND OF THE INVENTION

The exothermic Sabatier reaction produces methane and water during carbon dioxide and hydrogen reaction at stoichiometric ratio of 1:4, temperatures above 300-500 °C, and high pressures 1-10 MPa, and in the presence of a catalyst, usually Ni-based supported on aluminium oxide. The reaction is as follows:

$$CO_2 + 4H_2 \leftrightarrow CH_4 + 2H_2O, \Delta H = -165\frac{kJ}{mol}.$$

Beside Ni, other transition metals such as Ru, Rh, Pd and Pt are generally used as catalytic material for methanation in the form of metal nanoparticles (MNP) deposited on various supports (MNP/support). Also, new methanation catalyst support materials, such as metal oxides, modified oxides, metal-organic frameworks, zeolites, mesoporous silica, carbon materials, etc., have been widely investigated to increase its activity and stability. Reversible $CO_2$ hydrogenation reaction (Sabatier reaction) requires *in-situ* water removal to get higher methane yield and selectivity. Therefore, the introduction of water-selective materials (sorbents or membranes) have been widely studied. Among the different water-selective materials that have been proposed and tested in $CO_2$ hydrogenation literature, zeolite materials are the most widely used followed by silica, ceramics, etc. (Faria et al., 2018, https://doi.org/10.1016/j.jcou.2018.05.005). ASHOK JANGAM ET AL: "A review of recent catalyst advances in CO2 methanation processes",CATALYSIS TODAY, ELSEVIER, AMSTERDAM, NL, vol. 356, 29 July 2020 (2020-07-29), pages 471-489,discloses CO2 methanation processes. It states that the reaction can be performed using plasma including a gliding arc plasma and that Ni on a high surface area porous material is a suitable catalyst.

Exploration of novel catalysts and supports as well as demonstration of complete value chain and energy-efficient technology for carbon dioxide methanation has received much attention. Recently, plasma-assisted both catalytic and non-catalytic carbon dioxide hydrogenation has been applied to overcome the barriers of the traditional thermo-catalytic process. The use of plasma enables activation of molecules via excitation, dissociation and ionization, thus generating reactive species such as electrons, ions, radicals, exited species, etc. These reactive plasma species contribute to both the gas phase and surface reactions, undertaking new reaction pathways proceeding at ambient temperatures to 200 °C and atmospheric pressures to several bar.

Therefore, exists the need for synthetic methane production with *in-situ* water removal in which the process can be carried out at low temperatures and pressures, thus overcoming shortcomings typical for conventional thermal catalytic carbon dioxide hydrogenation process.

SUMMARY OF THE INVENTION

The novelty of the present invention relates to a method performing the gliding arc plasma-catalytic carbon dioxide hydrogenation with *in-situ* water removal and additional hydrogen production from it via plasma-activated aluminium and water/water vapour reaction at low temperatures and atmospheric to moderate-level pressures of 1-5 bars.

The present disclosure also relates to a system suitable for performing the process according to the present invention.

**BRIEF DESCRIPTION OF THE DRAWINGS**

Fig. 1 represents a scheme of a gliding arc discharge plasma (GADP) catalytic reactor for $CO_2$ hydrogenation.

Fig. 2 represents a multi-point BET plot of a high <u>specific</u> surface area <u>of 1012.6 m$^2$/g</u>

Fig. 3 shows X-ray diffraction (XRD) patterns of Ni/PCH catalyst.

Fig. 4 represents a scheme of reaction chamber for *in-situ* water/water vapour removal and additional hydrogen production.

Fig. 5 represents a plot related to additional hydrogen production via activated aluminium and water reaction during GADP catalytic $CO_2$ methanation process.

## DETAILED DESCRIPTION OF THE INVENTION

The present invention relates to a method and a system for performing the following Sabatier reaction (R1) using the synergetic effect of a gliding arc discharge plasma (GADP) and a catalyst with subsequent *in-situ* water removal and additional hydrogen production via water and plasma-activated aluminium reaction (R2) at low temperatures and atmospheric to moderate-level pressures:

$$CO_2 + 4H_2 \leftrightarrow CH_4 + 2H_2O \qquad (R1)$$

$$3H_2O + 2Al \rightarrow 3H_2 + Al_2O_3 \qquad (R2)$$

The method characterised in that:

- said catalyst is based on the combination of a high <u>specific</u> surface area porous clay heterostructure (PCH) support and nickel - Ni/PCH catalyst; and
- said a mixture of $CO_2$ and $H_2$ gases and a catalyst are activated by a gliding arc discharge plasma (GADP); and
- said produced water/water vapour is removed via reaction with plasma-activated aluminium (R2); and
- said additional hydrogen is produced to react with carbon dioxide thus minimizing the initial amount of hydrogen needed for the process.

The support of Ni-based catalyst is made of a porous high specific surface area ($S_{BET}$) and pore volumes clay heterostructure formed by chemical method.

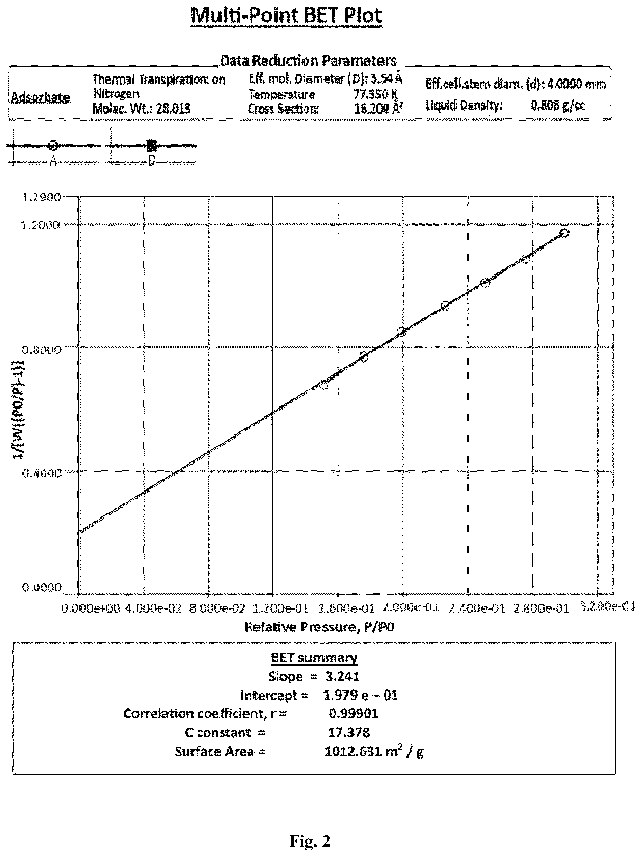

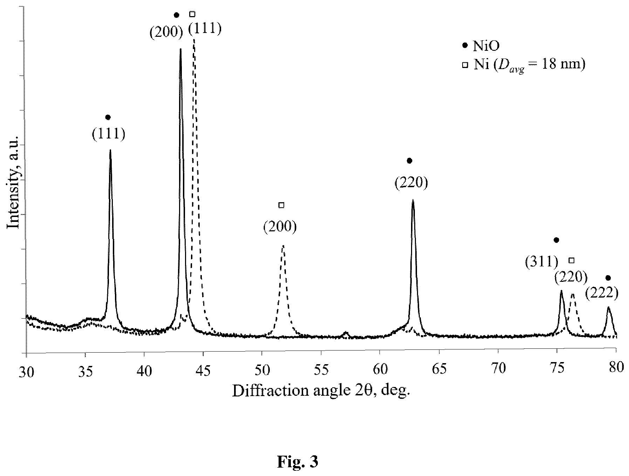

The support of the Ni/PCH catalyst has a high specific surface area $S_{BET}$ of 1000 to 1100 $m^2$/g, total pore volume of 1.15 $cm^3$/g, good dispersion of Ni nanoparticles $D_{avg}$ 18 nm. The multi-point BET specific surface area of the catalyst support was measured using the gas sorption (physisorption/chemisorption) system: the Quantachrome Autosorb iQ instrument and Quantachrome's ASiQwin Software. Nitrogen $N_2$ was used as the adsorbent gas. The total pore volume is derived from the amount of $N_2$ vapor adsorbed at a relative pressure $P/P_0$ close to unity, by assuming that the pores are then filled with liquid $N_2$ adsorbate. The high specific surface area of support encourages a better dispersion of Ni nanoparticles, improves the formation of active centres, and has good availability of gaseous reactants and higher conversion as well as catalytic activity. Fig. 2 shows the multi-point BET plot of a high specific surface area of 1012.6 $m^2$/g porous clay heterostructure support used for Ni catalyst. Fig. 3 shows XRD pattern of the distribution on Ni nanoparticples on the PCH support. The average diameter of the Ni nanoparticles was measured using the X-ray diffraction method, TOPAS 4.1 software and the Scherrer equation.

The Ni loading varies from 5 to 30 wt% on the PCH catalyst support. Preferably, the higher the Ni loading, the better performance of the Ni/PCH catalyst is achieved. Table 1 shows evaluation of Ni/PCH catalyst efficiency increasing the Ni loading from 5 to 30 wt%.

Table 1

| No. | Ni loading wt% | Conversion ($X_{CO2}$), % | Selectivity ($S_{CH4}$), % | Yield ($Y_{CH4}$), % |
| --- | --- | --- | --- | --- |
| 1 | 5 | 18.6 | 18.6 | 3.44 |
| 2 | 10 | 28.6 | 92.0 | 26.4 |
| 3 | 20 | 80.0 | 75.0 | 60.3 |
| 4 | 30 | 94.0 | 94.0 | 88.6 |

Process conditions: fixed-bed tube reactor, stoichiometric $H_2$:$CO_2$ ratio 4:1, 10 bars pressure, average temperature 400 °C, and gas hourly space velocity (GHSV) 756 1/h.

The average size of the Ni/PCH catalyst is 3 mm in diameter and 4-5 mm in length. The Ni/PCH catalyst during conventional thermo-catalytic carbon dioxide methanation process is characterized by $CO_2$ conversion of 94%, synthetic $CH_4$ yield of 88.6% and selectivity of 94%, respectively. Process conditions: fixed-bed tube reactor, stoichiometric $H_2:CO_2$ ratio 4:1, 10 bars pressure, average 400 °C temperature, and gas hourly space velocity (GHSV) of 756 1/h.

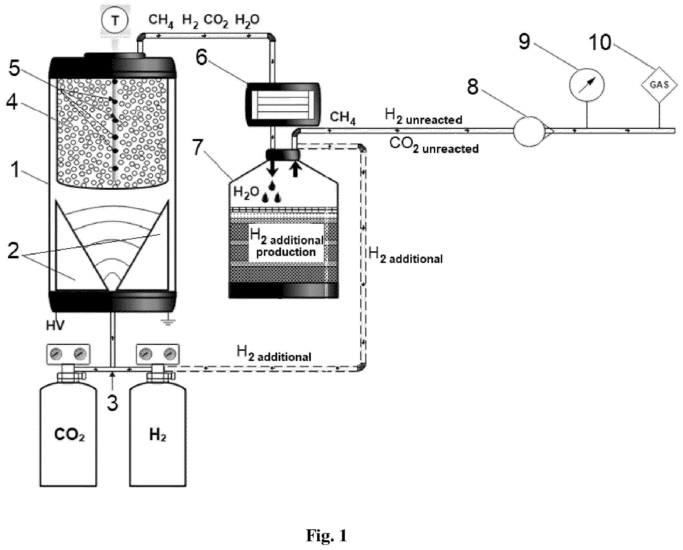

The Ni/PCH catalyst is reduced under hydrogen flow at 480 °C for 2 hours. The reduced Ni/PCH catalyst is then placed over the surface of the electrodes 2 of the gliding arc discharge plasma (GADP) in the reactor (Fig. 1). The distance between the electrodes 2 and the catalyst bed 4 is in the range of 1-2 cm. The catalyst is not directly immersed into the GADP due to too high thermal load, potential physical damage and deactivation.

A mixture of gases $CO_2$ and $H_2$ 3 is activated, i.e. ionized by a GADP then passes through the placed catalyst 4 thus initiating adsorption-desorption catalyst-gas phase interaction reactions.

Gliding arc discharge plasma is used to operate as the main discharge source as it shares both properties typical for thermal and non-thermal plasmas and is suitable for $CO_2$ conversion.

No external heating is used in the process. GADP is a source of heat. Preferably, the process is carried out at atmospheric to moderate-level up to 5 bars, absolute up to 6 bars pressure.

Synergy of the GADP activating $CO_2$ and $H_2$ gases via vibrational excitation (different activation mechanism compared to a direct barrier discharge (DBD)) and later activated gas mixture interaction with catalyst via heterogeneous adsorption/desorption reactions to produce ultimate desired product synthetic methane is one of the key novelty of the present invention.

No other gases, except $CO_2$ and $H_2$, are used to form plasma.

A reactor suitable for carrying out plasma-assisted catalytic carbon dioxide hydrogenation process according to the present invention is disclosed in Fig. 1. The reactor according to Fig. 1, which is used only with illustrative purposes, comprises the following parts:

i. A cylindrical quartz tube 1, which is the reactor wall. The external part of the tube 1 is covered by a 2 mm thick perforated stainless steel tube to protect the reactor from fracture while working at moderate-level pressures.

ii. At the bottom of the quartz tube 1, two electrodes 2, made of stainless steel, are placed to generate plasma. The distance between the electrodes 2 can vary from 1-2 mm. The length of the electrodes 2 is 20 mm and the width is 10 mm. The electrodes have the shape of sails. The electrodes are insulated by a Teflon ring.

iii. Plasma-forming feed gas ($CO_2$ and $H_2$) is supplied via a 2 mm diameter tube 3 installed at the very bottom of the reactor.

iv. The catalyst bed 4 is placed over the electrodes 2 at the distance from 1-2 cm.

v. A K-type thermocouple temperature profile probe 5 is placed axially the reactor length in the centre to measure temperature profile every 10 mm at six different points.

vi. A condenser 6 is used to condense reaction by-product water.

vii. A stainless steel water removal reaction chamber 7, containing plasma-activated aluminium, is used to remove reaction water and produce additional hydrogen via plasma-activated aluminium and water reaction.

viii. A rotameter 8 is used to measure the flow rate of gaseous reaction products.

ix. A pressure control valve 9.

x. Gas chromatograph (GC) and gas analyser 10.

The high voltage electrodes 2 can be supplied by an AC or DC or pulsed current with a voltage of 10 kV (1.5-3.0 kV, 30-50 mA, current conditions). The power injected in these conditions is in the range of 100-140 W.

The ratio of feed gas $H_2$ and $CO_2$ is 1.66, i.e. 10 l/min $H_2$ and 6 l/min $CO_2$. The total flow of 16 l/min is used as a plasma-forming gas and a reaction gas.

Gaseous products composition is measured by gas chromatography and gas analysis methods.

The condensable products, mainly water vapour, are collected in a chamber 7 for *in situ* water removal and additional hydrogen production to be used again as a reactant in the Sabatier process. Whereas oxygen is captured in the reaction by-products. This is the second key novelty of the present invention minimizing the need of hydrogen for $CO_2$ methanation process:

I. Aluminium scrap is activated in a glow discharge plasma in the presence of 3-10 Pa of hydrogen with purity not less than 99.00%, or argon with purity not less than 99.00%, or hydrogen and argon mixtures with the best Ar:$H_2$ proportion 70:30 as residual gasses for 10-60 min treatment in plasma with the DC power density up to 10-30 W/cm$^2$. Temperature is 60-110 $^0$C during the plasma process.

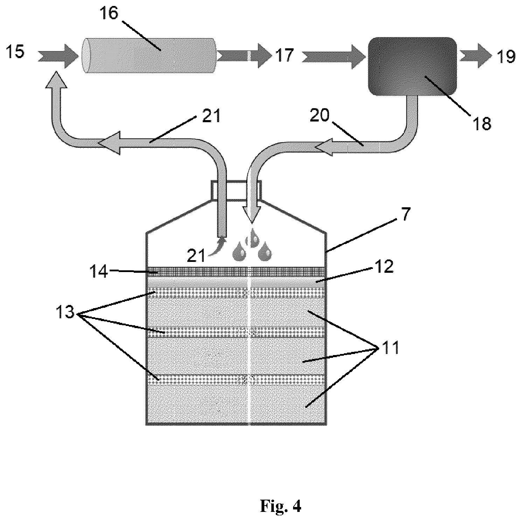

II. 30 g of plasma-activated hydrophilic aluminium 11 is placed in a chamber 7 (Fig. 4) in a layered structure between the layers of NaOH particles 13 (Fig. 4). The proportion of layers thickness between the layers of NaOH particles 13, acting as a reaction promoter between plasma-activated aluminium and water molecules, and activated aluminium layer 11 is 1:5 by volume. A stainless steel mesh 14 with pores diameters of 1 mm and glass wool 12 were used at the inlet and outlet of reaction molecules to prevent aluminium and reaction by-products Bayerite - $Al(OH)_3$, thermonatrite

- $Na_2CO_3 \cdot H_2O$ migration from the reaction chamber 7.

III. In case a water condensation equipment 6 is present (Fig. 1), $H_2O$ molecules enter the chamber 7 were reaction between activated aluminium 11 and condensed water molecules (R2 reaction) takes place and pure $H_2$ gas and reaction by-products Bayerite - $Al(OH)_3$, thermonatrite - $Na_2CO_3 \cdot H_2O$ are produced. The other gases, such as $CH_4$, $CO_{2\,unreacted}$ and $H_{2\,unreacted}$, also entering the chamber 7, have no impact on reaction between activated aluminium and water. They just leave the chamber and go for analysis.

**EXAMPLES**

The present invention is further disclosed by way of examples which only intend to illustrate the present invention and by no means are limiting the scope thereof.

**Example 1**

A typical reactor operation and procedure as disclosed in the present invention is as follows:

1. With 25 g of Ni/PCH catalyst with 30 wt% Ni loading corresponding to an GHSV of 7700 1/h, the reactor, shown in Fig. 1, is supplied with a mixture of $CO_2$ and $H_2$ with a flow rate of 6 l/min and 10 l/min, respectively, at atmospheric to 4 bars absolute pressure and at temperature of 20 °C. Distance from the GADP electrodes 2 to the catalyst bed 4 is 1 cm. The parameters of the plasma generator: current - 1.5 A, voltage - 300 V, frequency - 250 kHz. The parameters of the GADP: current 40-50 mA, voltage 2.5-3.5 kV.

2. Without the GADP and presence of Ni/PCH catalyst at atmospheric and increased pressure to 4 bars, no change in the composition of outlet gases is observed. When the GADP is switched on, the temperature increases slowly and after 2 minutes the production of methane and water vapour is obtained at around 60-80 °C and atmospheric pressure, which is impossible to obtain in conventional thermo-catalytic Sabatier reaction.

3. With the GADP switched on and absence of Ni/PCH catalyst, no methane is obtained. Plasma activates and converts only $CO_2$ to CO and $H_2$ to H.

4. The steady state conditions producing methane are reached after around 7 minutes.

5. The temperature in the reactor is not controlled, i.e. the reactor is not cooled, and therefore the temperature profile measured by the K-type thermocouple in the reaction zone at six different places 5 (Fig. 1), starting from the hottest zone close to the GADP electrodes 2 and the coolest zone at the outlet of the reactor, settles down at 550°C, 380 °C, 310 °C, 240 °C, 150 °C, and 110°C, respectively.

6. Such a high temperature is reached due to exothermal nature of the catalytic R1 reaction.

7. The temperature profile in the reactor measured without the catalyst and the GADP switched on at the same experimental conditions is as follows: 315 °C, 280 °C, 260 °C, 240 °C, 220 °C, and 180 °C, respectively.

8. The efficiency of the GADP catalytic process for $CO_2$ methanation is summarized in Table 2.

Table 2

| Pressure in reactor, bar | Ni/PCH catalyst with 30 wt% Ni loading | | | | | | | | |
|---|---|---|---|---|---|---|---|---|---|
| | $X_{CO2}$, % | $X_{H2}$, % | $CH_4$, vol% | $CH_4^T$, vol% | $S_{CH4}$, % | $Y_{CH4}$, % | $CB^1$, % | $SER^2$, kWh/m³ | SER, kJ/mol |
| 1 | 22.29 | 48.01 | 6.4 | 21.9 | 18.99 | 4.23 | 88.98 | 3.06 | 246.21 |
| 2 | 26.41 | 52.87 | 8.3 | 22.0 | 20.21 | 5.34 | 87.34 | 2.43 | 195.30 |
| 3 | 26.07 | 55.01 | 11.5 | 22.0 | 28.03 | 7.31 | 89.77 | 1.77 | 142.66 |
| 4 | 27.09 | 54.01 | 13.3 | 22.0 | 33.27 | 9.02 | 89.01 | 1.26 | 101.16 |
| $^T CH_4$ - methane concentration at thermodynamic equilibrium $^1$CB - carbon balance $^2$SER - specific energy requirements | | | | | | | | | |

9. In all cases the production of methane starts after 2 minutes at 60-80 °C temperature. The GADP activates both $CO_2$ and $H_2$ gases via vibrational excitation as well as minimizes the activation barrier of Ni/PCH catalyst thus making adsorption/desorption of gases on its surface possible at such low temperature.

10. Pressure has a positive impact on higher conversion of $CO_2$ and $CH_4$ selectivity and yield. However, the reaction in the present invention proceeds at atmospheric pressure and low temperature as conventional $CO_2$ methanation (Sabatier) reaction is limited to such conditions.

11. The specific energy requirements (SER) are in the range of 3.46 to 8.42 kWh/m³, or 278.19 to 677.07 kJ/mol. This is mostly depended on a higher $CO_2$ and $H_2$ conversion and $CH_4$ concentration at increased pressure.

12. High value of carbon balance around 89% show that carbon deposition is minimal to clog catalyst pores thus reducing its efficiency. No solid carbon deposition on catalyst surface is observed.

**Example 2**

Another GADP catalytic reactor operation and procedure as disclosed in the present invention is as follows:

1. With 25 g of Ni/PCH catalyst with 30 wt% Ni loading corresponding to an GHSV of 7700 1/h, the reactor is supplied with a mixture of $CO_2$ and $H_2$ with a flow rate of 6 l/min and 10 l/min, respectively, at atmospheric to 4 bars absolute pressure at temperature of 20 °C. Distance from the GADP electrodes 2 to the catalyst bed 4 is 1 cm. The parameters of the plasma generator: current - 1.5 A, voltage - 300 V, frequency - 250 kHz. The parameters of the GADP: current 40-50 mA, voltage 2.5-3.5 kV. 30 g of plasma-activated hydrophilic aluminium is placed in the developed innovative chamber to react with condensed water.

2. *In situ* water removal with subsequent additional hydrogen production via R2 reaction is carried out. Fig. 4 shows how the water is removed and additional hydrogen is produced during plasma-catalytic carbon dioxide hydrogenation (15 - reaction gases: $CO_2$ + $H_2$, 16 - GADP catalytic reactor (or traditional Sabatier reactor), 17 - product gases: $CH_4$ + $H_2O$, plus unreacted $CO_2$ and $H_2$, 18 - condenser, 19 - product gases without water: CH4, plus unreacted $CO_2$ and $H_2$, 20 - condensed water, 7 - chamber, 14 - stainless steel mesh, 12 - glass wool, 13 - NaOH, 11 - plasma-activated hydrophilic aluminium, 21 - additional hydrogen production). A mixture of gases ($CH_{4\,produced}$, $CO_2$ and $H_{2\,unreacted}$), in case of entering the chamber 7, has no direct impact on additional hydrogen production. They simply leave the chamber having no direct impact on condensed water and activated aluminium reaction to produce additional hydrogen.

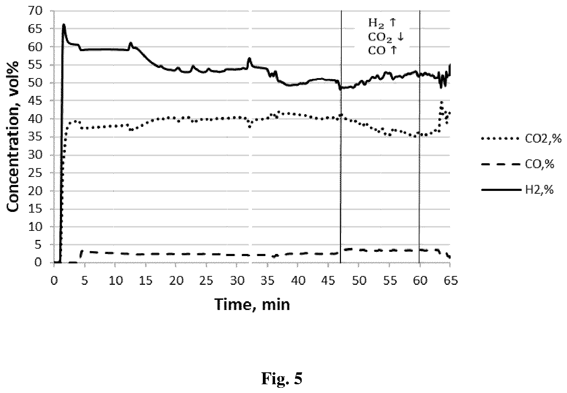

3. Fig. 5 shows additional $H_2$ production according to R2 reaction. Additional $H_2$ production starts after 47 minutes and ends after 60 minutes of the continuous operation of the process producing methane. The concentration of product gases is measured by a gas analyser.

4. During this process, beside the reaction between condensed water and activated aluminium (R2 reaction), a reversible water-gas shift (RWGS) reaction also dominates. According to RWGS the concentration of $H_2$ and $CO_2$ should decrease, and the one of CO and $H_2O$ should increase. However, $H_2$ concentration increased up to 5-6 vol% ($CO_2$ decreased up to 5 vol%, CO increased up to 2-3%) implying the presence of the R2 reaction.

5. Produced additional hydrogen could be used as a reactant in $CO_2$ methanation process, thus minimizing the need of green hydrogen needed for the reaction.

**Claims**

1.    A method for performing the reactions (R1), (R2) with catalyst and *in-situ* water/water vapour removal and additional hydrogen production:

$$CO_2 + 4H_2 \leftrightarrow CH_4 + 2H_2O \qquad (R1)$$

$$3H_2O + 2Al \rightarrow 3H_2 + Al_2O_3 \qquad (R2)$$

the method **characterised in that:**

- the catalyst is based on the combination of a high specific surface area of 1000 to 1100 $m^2$/g, measured by a gas sorption method with $N_2$ gas as adsorbent, porous clay heterostructure (PCH) support and nickel - Ni/PCH catalyst; and
- a mixture of $CO_2$ and $H_2$ gases and a catalyst is activated by a gliding arc discharge plasma (GADP) ) to further perform (R1); and
- the produced reaction by-product water/water vapour is *in-situ* removed via reaction with plasma-activated aluminium (R2); and
- as the result of the R2 reaction, the additional hydrogen is produced to react with carbon dioxide, thus minimizing the initial amount of hydrogen needed for the process.

2.    The method according to claim 1, **characterized in that** the catalyst is based on the combination of nickel and a high specific surface area porous clay heterostructure (PCH) as a support formed by a chemical method.

3. The method according to claim 1, **characterized in that** the Ni/PCH catalyst comprises nickel of 5 to 30 wt%, most preferably 30 wt%, and a PCH support of specific surface area of 1000 to 1100 $m^2$/g, and total pore volume up to 1.15 $cm^3$/g, measured by a gas sorption method with $N_2$ adsorbent, and dispersion of Ni nanoparticles ($D_{avg}$ 18 nm) defined using the X-ray diffraction method.

4. The method according to claim 1, **characterized in that** the Ni/PCH catalyst during the conventional thermo-catalytic carbon dioxide methanation process in a fixed-bed tube reactor at the $H_2$:$CO_2$ ratio of 4:1, reactor pressure of 10 bars, average temperature of 400 °C, GHSV of 756 l/h, and Ni loading 30 wt.%, is **characterized by** $CO_2$ conversion of 94.0%, $CH_4$ yield of 88.6%, and selectivity of 94%, respectively.

5. The method according to claim 1, **characterized in that** the gliding arc discharge plasma (GADP) is used to activate carbon dioxide and hydrogen via vibrational excitation pathway and active sites of Ni/PCH catalyst at atmospheric to moderate pressures up to 5 bars and low temperatures to produce synthetic methane.

6. The method according to claim 1 or 5, **characterized in that** no other gases, except $CO_2$ and $H_2$, are used to form plasma and active medium of gas mixture to interact with catalyst via heterogeneous adsorption/desorption reactions to produce synthetic methane and water.

7. The method according to any of claims 1 to 6, **characterized in that** the hydrogen and carbon dioxide are provided in an $H_2$/$CO_2$ ratio of 1.66, i.e. 10 l/min $H_2$ and 6 l/min $CO_2$.

8. The method according to claim 1 to 7, **characterized in that** the synthetic methane production, according to R1 reaction, starts preferably at 60 to 80 °C temperature, most preferably at 60 °C temperature, and atmospheric to moderate-level pressure up to 5 bars, most preferably atmospheric pressure, and after two minutes of plasma ignition.

9. The method according to claim 1 to 8, **characterized in that** the water molecules, produced after R1 reaction, react with activated aluminium (R2 reaction) instantly after entering the reaction zone and are converted to pure hydrogen, and oxygen is captured in reaction by-products Bayerite - $Al(OH)_3$ and $Na_2CO_3{\cdot}H_2O$ thermonatrite.

10. A system for producing synthetic methane from carbon dioxide and hydrogen with *in-situ* water removal and additional hydrogen production **characterised by** comprising a gliding arc discharge (GAD) device arranged to generate plasma; a catalyst comprising nickel on a support comprising porous clay heterostructure with a high specific surface area of 1000 to 1100 $m^2$/g measured by a gas sorption method with $N_2$ adsorbent; a reaction chamber, containing plasma-activated aluminium, for *in-situ* water/water vapour removal and additional hydrogen production.

**Patentansprüche**

1. Verfahren zum Ausführen der Reaktionen (R1), (R2) mit einem Katalysator und In-Situ-Wasser-/Wasserdampfentfernung und zusätzlicher Wasserstofferzeugung:

$$CO_2 + 4H_2 \leftrightarrow CH_4 + 2H_2O \qquad (R1)$$

$$3H_2O + 2Al \rightarrow 3H_2 + Al_2O_3 \qquad (R2)$$

wobei das Verfahren **dadurch gekennzeichnet ist, dass:**

- der Katalysator auf der Kombination eines porösen Tonheterostruktur-(PCH) Trägers hohen spezifischen Oberflächenbereichs von 1000 bis 1100 $m^2$/g, durch ein Gassorptionsverfahren mit $N_2$-Gas als Adsorptionsmittel gemessen, und Nickel-Ni/PCH-Katalysators basiert; und
- eine Mischung von $CO_2$- und $H_2$-Gasen und einem Katalysator durch ein gleitendes Bogenentladungsplasma (GADP) aktiviert wird, um (R1) noch weiter auszuführen; und
- das erzeugte Reaktionsnebenprodukt von Wasser/Wasserdampf in situ durch Reaktion mit plasmaaktiviertem Aluminium (R2) entfernt wird; und
- als Ergebnis der R2-Reaktion der zusätzliche Wasserstoff zum Reagieren mit Kohlendioxid erzeugt wird, um so die anfängliche Menge Wasserstoff, die für den Vorgang erforderlich ist, zu minimieren.

2. Verfahren nach Anspruch 1, **dadurch gekennzeichnet, dass** der Katalysator auf der Kombination von Nickel und einer porösen Tonheterostruktur (PCH) von hohem spezifischem Oberflächenbereich als durch ein chemisches Verfahren gebildetem Träger basiert.

3. Verfahren nach Anspruch 1, **dadurch gekennzeichnet, dass** der Ni/PCH-Katalysator Nickel von 5 bis 30 Gew.-%, am bevorzugtesten 30 Gew.-%, und einen PCH-Träger eines spezifischem Oberflächenbereichs von 1000 bis 1100 $m^2$/g und eines gesamten Porenvolumens von bis zu 1,15 $cm^3$/g, durch ein Gassorptionsverfahren mit $N_2$-Adsorptiopnsmittel gemessen, und einer Dispersion von Ni-Nanopartikeln ($D_{duchschn}$. 18 nm) unter Anwendung des Röntgenbeugungsverfahrens definiert, umfasst.

4. Verfahren nach Anspruch 1, **dadurch gekennzeichnet, dass** der Ni/PCH-Katalysator während des herkömmlichen thermokatalytischen Kohlendioxidmethanisierungsvorgangs in einem Festbettröhrenreaktor bei dem $H_2$:$CO_2$-Verhältnis von 4:1, einem Reaktordruck von 10 bar, einer Durchschnittstemperatur von 400 °C, einer stündlichen Gasraumgeschwindigkeit von 756 l/h und einer Ni-Ladung von 30 Gew.-% jeweils durch eine $CO_2$-Umwandlung von 94,0 %, einen $CH_4$-Ertrag von 88,6 % und eine Selektivität von 94 % gekennzeichnet ist.

5. Verfahren nach Anspruch 1, **dadurch gekennzeichnet, dass** gleitende Bogenentladungsplasma (GADP) verwendet wird, Kohlendioxid und Wasserstoff über einen Vibrationsanregungsweg und aktive Orte von Ni/PCH-Katalysator bei Luft- bis mittlerem Druck von bis zu 5 bar und niederen Temperaturen zu aktivieren, um synthetisches Methan herzustellen.

6. Verfahren nach Anspruch 1 oder 5, **dadurch gekennzeichnet, dass** keine anderen Gase außer $CO_2$ und $H_2$ verwendet werden, um Plasma und aktives Medium von einer Gasmischung zu bilden, um mit Katalysator über heterogene Adsorptions-/Desorptionsreaktionen zu interagieren, um synthetisches Methan und Wasser herzustellen.

7. Verfahren nach einem der Ansprüche 1 bis 6, **dadurch gekennzeichnet, dass** der Wasserstoff und Kohlendioxid in einem $H_2$/$CO_2$-Verhältnis von 1,66, d.h. 10 l/min $H_2$ und 6 l/min $CO_2$ bereitgestellt werden.

8. Verfahren nach Anspruch 1 bis 7, **dadurch gekennzeichnet, dass** die Herstellung von synthetischem Methan der R1-Reaktion entsprechend bevorzugt bei einer Temperatur vom 60 bis 80 °C, am bevorzugtesten einer Temperatur von 60 °C und bei Luft- bis mäßigen Druck von bis zu 5 bar, am bevorzugtesten Luftdruck und nach zwei Minuten Plasmaentzündung beginnt.

9. Verfahren nach Anspruch 1 bis 8, **dadurch gekennzeichnet, dass** die Wassermoleküle, die nach der R1-Reaktion erzeugt werden, mit aktiviertem Aluminium (R2-Reaktion) sofort nach Eingang in die Reaktionszone reagieren und in reinen Wasserstoff umgewandelt werden und Sauerstoff in den Reaktionsnebenprodukten Bayerit - Al(OH)$_3$ und Na$_2$CO$_3$•H$_2$O-Thermonatrit - aufgefangen wird.

10. System für die Herstellung von synthetischem Methan aus Kohlendioxid und Wasserstoff mit In-Situ-Wasserentfernung und zusätzlicher Wasserstofferzeugung, **gekennzeichnet durch** Umfassen einer gleitenden Bogenentladungs- (GAD) Vorrichtung, die zum Erzeugen von Plasma angeordnet ist; eines Katalysators, der Nickel auf einem Träger umfasst, der eine poröse Tonheterostruktur mit einem hohen spezifischen Oberflächenbereich von 1000 bis 1100 $m^2$/g, durch ein Gassorptionsverfahren mit $N_2$-Gas gemessen, umfasst; einer Reaktionskammer, die plasmaaktiviertes Aluminium enthält, zur In-Situ-Wasser/Wasserdampfentfernung und zusätzlichen Wasserstofferzeugung.

**Revendications**

1. Procédé de réalisation des réactions (R1), (R2) avec un catalyseur et enlèvement de la vapeur eau/eau *in situ* et production d'hydrogène supplémentaire :

$$CO_2 - 4H_2 \rightarrow CH_4 + 2H_2O \qquad (R1)$$

$$3H_2O + 2Al \rightarrow 3H_2 - Al_2O_3 \qquad (R2)$$

le procédé **caractérisé en ce que** :

- le catalyseur est basé sur la combinaison d'une surface hautement spécifique de 1 000 à 1 100 m$^2$/g, mesurée par un procédé de sorption de gaz avec le gaz $N_2$ comme adsorbant, un support d'hétérostructure d'argile poreuse (PCH) et de catalyseur nickel Ni/PCH ; et

- un mélange de gaz $CO_2$ et $H_2$ et un catalyseur est activé par un plasma de décharge d'arc de glissement (GADP) pour encore effectuer (R1) ; et

- la vapeur d'eau/eau de sous-produit de réaction produite est enlevée *in situ* via la réaction avec de l'aluminium activé par plasma (R2) ; et

- suite à la réaction R2, l'hydrogène supplémentaire est produit pour réagir avec le dioxyde de carbone, minimisant ainsi la quantité initiale d'hydrogène nécessaire pour le processus.

2. Procédé selon la revendication 1, **caractérisé en ce que** le catalyseur est basé sur la combinaison de nickel et d'une hétérostructure d'argile poreuse (PCH) de surface hautement spécifique comme support formé par un procédé chimique.

3. Procédé selon la revendication 1, **caractérisé en ce que** le catalyseur Ni/PCH comprend du nickel de 5 à 30 % en poids, de préférence entre toutes de 30 % en poids et un support PCH de surface spécifique de 1 000 à 1 100 m$^2$/g et un volume de pore total jusqu'à 1,15 cm$^3$/g, mesuré par un procédé de sorption de gaz avec l'adsorbant $N_2$ et la dispersion de nanoparticules de Ni ($D_{ang}$ 18 nm) défini en utilisant le procédé de diffraction aux rayons X.

4. Procédé selon la revendication 1, **caractérisé en ce que** le catalyseur Ni/PCH durant le processus de méthanation de dixoyde de carbone thermo-catalytique conventionnel dans un réacteur en tube à lit fixe au rapport $H_2$ : $CO_2$ de 4 : 1, une pression de réacteur de 10 bars, une température moyenne de 400 °C, un GHSV de 756 l/h et une charge de Ni de 30 % en poids, est **caractérisé par** une conversion de $CO_2$ de 94,0 %, un rendement de $CH_4$ de 88,6 % et une sélectivité de 94 %, respectivement.

5. Procédé selon la revendication 1, **caractérisé en ce que** le plasma de décharge d'arc de glissement (GADP) est utilisé pour activer le dioxyde de carbone et l'hydrogène via un chemin d'excitation vibrationnelle et des sites actifs de catalyseur Ni/PCH à des pressions atmosphérique à modérées de jusqu'à 5 bars et de basses températures pour produire le méthane synthétique.

6. Procédé selon la revendication 1 ou 5, **caractérisé en ce qu'**aucun autre gaz, à part le $CO_2$ et $H_2$, ne sont utilisés pour former le plasma et le milieu actif de mélange de gaz pour interagir avec le catalyseur via des réactions d'adsorption/désorption hétérogènes pour produire le méthane synthétique et l'eau.

7. Procédé selon l'une quelconque des revendications 1 à 6, **caractérisé en ce que** l'hydrogène et le dioxyde de carbone sont fournis dans un rapport $H_2$/$CO_2$ de 1,66, c'est-à-dire 10 l/min de $H_2$ et 6 l/min de $CO_2$.

8. Procédé selon les revendications 1 à 7, **caractérisé en ce que** la production de méthane synthétique, selon la réaction R1, débute de préférence à une température de 60 à 80 °C, de préférence entre toutes à une température de 60 °C et une pression de niveau atmosphérique à modéré jusqu'à 5 bars, de préférence entre toutes à pression atmosphérique et après deux minutes d'ignition de plasma.

9. Procédé selon les revendications 1 à 8, **caractérisé en ce que** les molécules d'eau, produites après la réaction R1, réagissent avec l'aluminium activé (réaction R2) instantanément après être entrées dans la zone de réaction et sont converties en hydrogène pur et l'oxygène est capturé dans les sous-produits de réaction bayérite Al(OH)$_3$ et thermonarrite Na$_2$CO$_3$•H$_2$O.

10. Système de production de méthane synthétique à partir de dioxyde de carbone et d'hydrogène avec enlèvement d'eau *in situ* et production d'hydrogène supplémentaire **caractérisé comme** comprenant un dispositif de décharge d'arc de glissement (GAD) disposé pour produire un plasma ; un catalyseur comprenant du nickel sur un support comprenant une hétérostructure d'argile poreuse avec une surface hautement spécifique de 1 000 à 1 100 m$^2$/g mesurée par un procédé de sorption de gaz avec l'adsorbant $N_2$, contenant de l'aluminium activé par plasma pour l'enlèvement de vapeur d'eau/eau *in situ* et production d'hydrogène supplémentaire.

**Fig. 1**

## Multi-Point BET Plot

Fig. 2

**Fig. 3**

Fig. 4

**Fig. 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• A review of recent catalyst advances in CO2 methanation processes. **ASHOK JANGAM et al.** CATALYSIS TODAY. ELSEVIER, 29 July 2020, vol. 356, 471-489